# EUROPEAN PATENT APPLICATION

(11) **EP 4 137 817 A1**
(43) Date of publication of application: **22.02.2023**
(21) Application number: 21788993.0
(22) Date of filing: 16.04.2021
(51) Int. Cl.: G01N 33/579, G01N 27/327, G01N 27/416

(54) **TRACE MATERIAL MEASUREMENT METHOD USING HYDROLASE**

(30) Priority: 16.04.2020 JP 2020073489
(71) Applicant: Kikkoman Corporation, Noda-shi, Chiba 278-8601 (JP)
(72) Inventor: TOTSUKA Naoya, Noda-shi, Chiba 278-8601 (JP); MASAKARI Yosuke, Noda-shi, Chiba 278-8601 (JP); MATSUE Tomokazu, Sendai-shi, Miyagi 980-8577 (JP); INOUE Kumi, Sendai-shi, Miyagi 980-8577 (JP); ITO Takahiro, Sendai-shi, Miyagi 980-8577 (JP)
(74) Representative: J A Kemp LLP
(86) International application number: PCT/JP2021/015693
(87) International publication number: WO 2021/210669

(57) **Abstract**

This invention provides a method of electrochemical measurement capable of detection or quantification of a trace substance in a simple manner. Such method of electrochemical measurement comprises using a substrate-label compound labeled with a label, wherein the label is used to convert a substrate into a substrate-label compound suitable for electrochemical measurement by binding to the substrate and wherein the label is capable of being adsorbed onto an electrode.

## Description

### Technical Field

The present disclosure relates to a method for electrochemical measurement of a trace substance using a hydrolase.

### Background Art

When a target substance is subjected to qualitative or quantitative measurement via electrochemical measurement using an enzyme, an oxidoreductase is generally used. In contrast, there are not many cases that use an enzyme other than an oxidoreductase, such as a hydrolase. In optical techniques, measurement involving the use of a hydrolase is common, and such technique is used for measurement of enzyme activity or measurement of endotoxin or (1→3)-β-D-glucan, which is a substance from a microbe (see, for example, Patent Literature 1).

Endotoxin is a lipopolysaccharide constituting the cell walls of Gram-negative bacteria. Endotoxin is a very stable substance and, therefore, it is difficult to eliminate or inactivate endotoxin if it ends up in the process of production and use of, for example, pharmaceutical products, medical equipment, and dialysis fluids. Endotoxin is a typical pyogen. If even a very small amount of endotoxin enters the blood due to administration of a substance contaminated with endotoxin, a fever is produced in a human or animal to which such substance has been administered and further, this is known to be the cause of highly lethal diseases, such as intravascular blood clotting associated with sepsis. In the fields of injection preparations, medical equipment, biopharmaceuticals, regenerative medical products, and dialysis agents, therefore, it is critical that products are endotoxin-free (i.e., pyrogen-free), and there is a need in the market for a technique that enables rapid and accurate detection of endotoxin contamination.

At present, the limulus test is a mainstream technique for endotoxin measurement. The limulus test is performed based on the reaction between an extract of horseshoe crab blood cells (the limulus amebocyte lysate, also referred to herein as "LAL") and endotoxin, and techniques, such as gelation, turbidimetry, colorimetry, and fluorometry, are known (see, for example, Patent Literature 1 and Patent Literature 2). Reagents for measurement using LAL are denoted as, for example, LAL reagents, lysate reagents, or limulus reagents, and such terms are synonymous with each other.

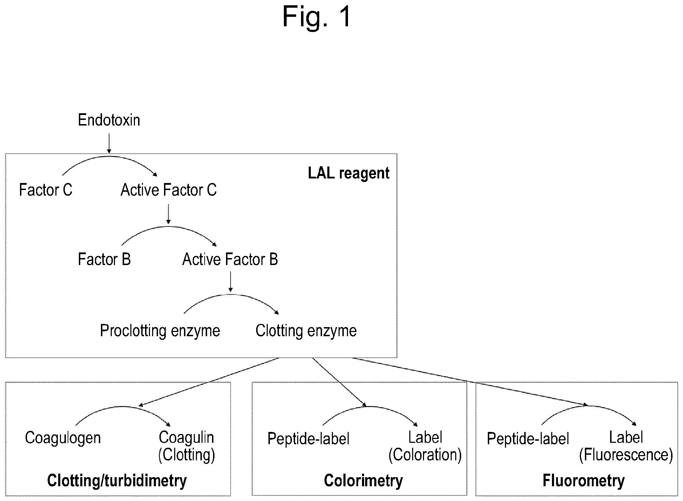

The principle of endotoxin measurement using LAL is as shown in a schematic diagram in Figure 1. Endotoxin converts Factor C as a proenzyme into an activated Factor C. Activated Factor C functions as a hydrolase and converts Factor B as a proenzyme into activated Factor B. Activated Factor B is also a hydrolase and converts Proclotting enzyme into activated clotting enzyme. The techniques of gelation and turbidimetry are methods in which activated clotting enzyme functions as a hydrolase and converts coagulogen in an LAL reagent into coagulative coagulin and the degree of this conversion is used as the indicator to measure endotoxin. In contrast, the technique of colorimetry is a method in which an activated clotting enzyme functions as a hydrolase and cleaves a bond between a peptide and a label of the label peptide designed for endotoxin measurement and the color development of the label as a result of such cleavage is assayed to measure endotoxin. The technique of fluorometry is a method in which a hydrolase in LAL acts on a label peptide designed for endotoxin measurement and a label is generated from the label peptide and fluorescence derived from the label is measured in order to measure endotoxin (see, for example, Patent Literature 2).

However, in gelation and turbidimetry, clotting that takes place in a sample is measured and, therefore, when a highly turbid specimen or colored specimen is measured, quantitative measurement may be difficult under certain conditions. In addition, measurement costs are relatively high in reality, because, for example, a relatively large amount of LAL reagents is used and special equipment is necessary for measurement. Colorimetry and fluorometry are problematic in terms of complicated procedures and a large amount of reagents required for measurement. Thus, a method that can detect a substance from a microbe, such as endotoxin, using a smaller amount of reagents via simple procedures is needed.

Under the above circumstances, attempts have been made to develop limulus test using electrochemical measurement. In particular, methods are gradually being proposed in which a label peptide is synthesized using an electrochemically active label, an activated clotting enzyme is allowed to react with the label peptide, and a label generated from the label peptide is electrochemically measured.

Non-Patent Literature 1 proposes a method for electrochemical measurement of endotoxin using a peptide to which para-aminophenol (p-AP) is bound. Patent Literature 3 proposes a method for quantitative measurement of endotoxin using a peptide to which para-methoxyaniline has bound, so as to overcome the problem of rapid loss in electrochemical activity of p-AP with the elapse of time. Patent Literature 4 proposes modification of an electrode structure so as to improve detection sensitivity of electrochemical measurement. Patent Literature 5 discloses a method of electrochemical measurement involving the use of a phenylenediamine derivative, such as N,N-dimethyl-p-phenylenediamine (DMPD).

However, little is known about techniques that enable electrochemical measurement of a trace substance, such as endotoxin, using hydrolase activity instead of oxidoreductases which, conventionally, were used widely. Therefore, the continuous development of technology is needed.

Trypsin is a type of a serine protease, and trypsin activity in the blood is generally measured in the field of biochemical testing for the purpose of, for example, diagnosis of pancreatic diseases. Further, gingipain is a type of a protease generated by a major pathogen of periodontal diseases; i.e., *Porphyromonas gingivalis,* and measurement of gingipain activity in the intraoral sample is applied to diagnosis of a periodontal disease of a subject. In addition, measurement of activity of hydrolases, such as alkaline phosphatase, esterase, and glycosidase, is common in various fields, including biochemical research and medicine, and development of a method that enables simple measurement of such enzyme activity is in need.

### Citation List

### Patent Literatures

Patent Literature 1: WO 95/14931
Patent Literature 2: JP Patent Publication (Kokai) No. 2009-150903
Patent Literature 3: JP Patent Publication (Kokai) No. 2015-187607
Patent Literature 4: JP Patent Publication (Kokai) No. 2018-072331
Patent Literature 5: WO 2017/141961

### Non-Patent Literatures

Non-Patent Literature 1: K. Y. Inoue et. al, Analyst, 2013, 138, 6523-6531

### Summary of Invention

### Technical Problem

An object of the present disclosure is to provide a novel electrochemical method for measuring a target using hydrolase activity.

### Solution to Problem

The present inventors have conducted concentrated studies in order to attain the above object. As a result, the present inventors discovered that a trace substance, such as endotoxin, can be measured in a simple manner by using a substrate-label compound obtained by binding a label, capable of being adsorbed onto an electrode, to a substrate that is designed for measurement, to electrochemically measure the label generated due to the reaction between a hydrolase and the substrate-label compound, thereby completing the present invention including such finding as an embodiment. The present inventors also discovered that trypsin and gingipain can be measured in a simple manner and completed the present invention including such finding as an embodiment.

The present disclosure encompasses the following embodiments.
[1] A method of electrochemical measurement comprising using a substrate-label compound labeled with a label, wherein the label is used to convert a substrate into a substrate-label compound suitable for electrochemical measurement by being bound to the substrate, and wherein the label is capable of being adsorbed onto an electrode.
[2] The method according to Embodiment 1, wherein the label is released from the substrate-label compound by the action of a hydrolase and the released label is measured electrochemically.
[3] The method of measurement according to Embodiment 1 or 2 for measuring the hydrolase activity of a specimen (analyte) or a substance that activates a hydrolase, which may be contained in the specimen.
[4] The method according to any one of Embodiments 1 to 3, wherein the substance that activates a hydrolase comprises endotoxin or (1→3)-β-D-glucan.
[5] The method according to any one of Embodiments 1 to 4, wherein the hydrolase is Factor C, Factor B, Factor G, and/or Proclotting enzyme of horseshoe crab, trypsin, gingipain, or phosphatase.
[6] The method according to any one of Embodiments 1 to 5, wherein the label is a phenylenediamine-type compound having a structure shown in Formula I: 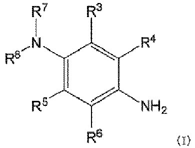 wherein
   R⁷ represents hydrogen or linear or branched C₁₋₆ alkyl, C₁₋₆ alkenyl, C₁₋₆ alkynyl, C₃₋₉ cycloalkyl, phenyl, 1-naphthyl, 2-naphthyl, anthracenyl, or phenanthrenyl, which may be optionally substituted with one or more X;
   R³, R⁴, R⁵, and R⁶ each independently represent hydrogen or linear or branched C₁₋₆ alkyl, C₁₋₆ alkenyl, C₁₋₆ alkynyl, C₁₋₆ alkoxy, halo, nitro, cyano, carboxy, sulfone, or amino, which may be optionally substituted with one or more Y; and
   R⁸ is selected from the group consisting of phenyl, 1-naphthyl, 2-naphthyl, anthracenyl, and phenanthrennyl, which may be optionally substituted with one or more X,
      wherein X represents linear or branched C₁₋₆ alkyl, C₁₋₆ alkenyl, C₁₋₆ alkynyl, C₁₋₆ alkoxy, anilino, phenoxy, halo, hydroxy, nitro, carboxy, cyano, sulfone, or amino, which may be optionally substituted with a substituent selected from the group consisting of halo, amino, cyano, carboxy, carbonyl, alkoxy, and sulfone, and
      Y is selected from the group consisting of halo, amino, cyano, carboxy, carbonyl, alkoxy, and sulfone, or
   an aminoanthraquinone-based compound having a structure shown in Formula II: 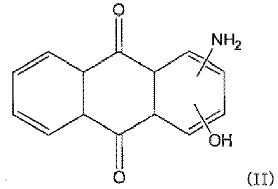
[7] The method according to Embodiment 6, wherein the phenylenediamine-type compound shown in Formula I is a compound selected from the group consisting of: 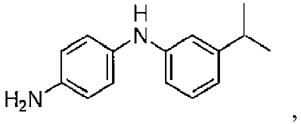 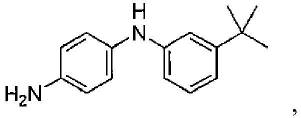 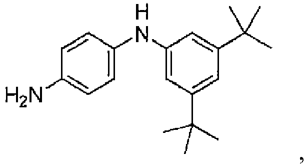 and 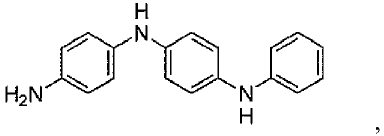 or
   the aminoanthraquinone-based compound shown in Formula (II) is 1-amino-4-hydroxyanthraquinone having a structure shown in the following formula: 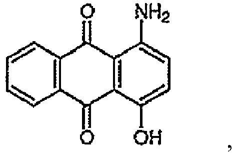
   2-amino-3-hydroxyanthraquinone having a structure shown in the following formula: 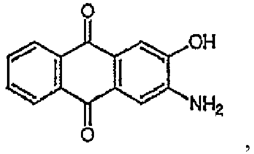 or
   a salt, an anhydride, or a solvate thereof.
[8] A reagent for electrochemical measurement of hydrolase activity or a substance that activates a hydrolase, said reagent comprising a substrate-label compound, wherein a label in the substrate-label compound is capable of being adsorbed onto an electrode.
[9] A composition for measurement of hydrolase activity or a substance that activates a hydrolase, said composition comprising the reagent according to Embodiment 8.
[10] An electrode comprising the reagent according to Embodiment 8 or the composition according to Embodiment 9.
[11] An electrochemical measurement system, wherein the electrode according to Embodiment 10 is used.
[12] The reagent according to Embodiment 8, the composition according to Embodiment 9, the electrode according to Embodiment 10, or the system according to Embodiment 11, wherein the label is a phenylenediamine-type compound having a structure shown in Formula I: 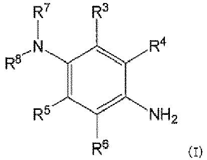 wherein
   R⁷ represents hydrogen or linear or branched C₁₋₆ alkyl, C₁₋₆ alkenyl, C₁₋₆ alkynyl, C₃₋₉ cycloalkyl, phenyl, 1-naphthyl, 2-naphthyl, anthracenyl, or phenanthrenyl, which may be optionally substituted with one or more X;
   R³, R⁴, R⁵, and R⁶ each independently represent hydrogen or linear or branched C₁₋₆ alkyl, C₁₋₆ alkenyl, C₁₋₆ alkynyl, C₁₋₆ alkoxy, halo, nitro, cyano, carboxy, sulfone, or amino, which may be optionally substituted with one or more Y; and
   R⁸ is selected from the group consisting of phenyl, 1-naphthyl, 2-naphthyl, anthracenyl, and phenanthrennyl, which may be optionally substituted with one or more X, wherein X represents linear or branched C₁₋₆ alkyl, C₁₋₆ alkenyl, C₁₋₆ alkynyl, C₁₋₆ alkoxy, anilino, phenoxy, halo, hydroxy, nitro, carboxy, cyano, sulfone, or amino, which may be optionally substituted with a substituent selected from the group consisting of halo, amino, cyano, carboxy, carbonyl, alkoxy, and sulfone, and Y is selected from the group consisting of halo, amino, cyano, carboxy, carbonyl, alkoxy, and sulfone, or
   an aminoanthraquinone-based compound having a structure shown in Formula II: 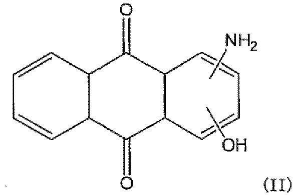
[13] The reagent, composition, electrode, or system according to Embodiment 12, wherein the phenylenediamine-type compound shown in Formula I is a compound selected from the group consisting of: 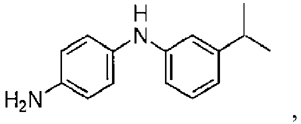 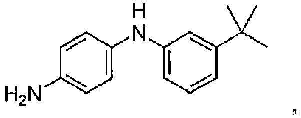 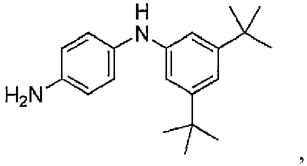 and 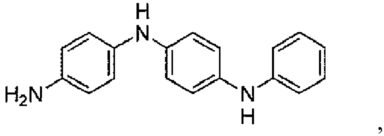 or
   the aminoanthraquinone-based compound shown in Formula (II) is 1-amino-4-hydroxyanthraquinone having a structure shown in the following formula: 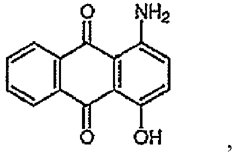
   2-amino-3-hydroxyanthraquinone having a structure shown in the following formula: 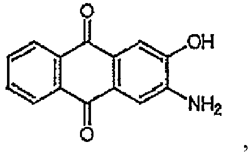 or
   a salt, an anhydride, or a solvate thereof.

The present specification encompasses the contents disclosed in Japanese Patent Application No. 2020-073489 on which the priority of the present application is based.

### Advantageous Effects of the Invention

The present disclosure enables stable measurement of hydrolase activity or a substance that activates a hydrolase.

### Brief Description of Drawings

[Figure 1] Figure 1 schematically shows the principle of a method of endotoxin measurement using LAL.
[Figure 2] Figure 2 shows the results of performing cyclic voltammetry using N-(3-isopropylphenyl)p-phenylenediamine and plotting a scan rate and IOmax.
[Figure 3] Figure 3 shows the results of performing cyclic voltammetry using N-(3-tert-butylphenyl)-p-phenylenediamine and plotting a scan rate and IOmax.
[Figure 4] Figure 4 shows the results of performing cyclic voltammetry using N-(3,5-di-tert-butylphenyl)-p-phenylenediamine and plotting a scan rate and IOmax.
[Figure 5] Figure 5 shows the results of performing cyclic voltammetry using N-(4-aminophenyl)-N'-phenyl-p-phenylenediamine and plotting a scan rate and IOmax.
[Figure 6] Figure 6 shows the results of performing cyclic voltammetry using p-aminophenol and plotting a scan rate and IOmax.
[Figure 7] Figure 7 shows the results of performing cyclic voltammetry using p-aminophenol and plotting a scan rate and IOmax. The horizontal axis depicts a square root of the scan rate.
[Figure 8] Figure 8 shows the relationship between the endotoxin concentration in the specimen and the oxidation current value at 120 mV when cyclic voltammetry is performed using a substrate-label compound in which Leu-Gly-Arg (a substrate) and N-(3-isopropylphenyl)p-phenylenediamine (a label) are bound to each other.
[Figure 9] Figure 9 shows the relationship between the endotoxin concentration in the specimen and the oxidation current value at 120 mV when cyclic voltammetry is performed using a substrate-label compound in which Leu-Gly-Arg (a substrate) and N-(3-tert-butylphenyl)-p-phenylenediamine (a label) are bound to each other.
[Figure 10] Figure 10 shows the relationship between the endotoxin concentration in the specimen and the oxidation current value at 110 mV when cyclic voltammetry is performed using a substrate-label compound in which Leu-Gly-Arg (a substrate) and N-(3,5-di-tert-butylphenyl)-p-phenylenediamine (a label) are bound to each other.
[Figure 11] Figure 11 shows the relationship between the endotoxin concentration in the specimen and the oxidation current value when chronoamperometry is performed using a substrate-label compound in which N-(3-isopropylphenyl)-p-phenylenediamine (a label) and Leu-Gly-Arg (a substrate) are bound to each other.
[Figure 12] Figure 12 shows results of cyclic voltammetry performed after a substrate-label compound in which N-(3-tert-butylphenyl)-p-phenylenediamine (a label) and Leu-Gly-Arg (a substrate) are bound to each other, is subjected to the reaction with solutions of trypsin adjusted at various concentrations.
[Figure 13] Figure 13 shows results of cyclic voltammetry performed after a substrate-label compound in which N-(3-tert-butylphenyl)-p-phenylenediamine (a label) and Val-Pro-Arg (a substrate) are bound to each other is subj ected to the reaction with the culture supernatant. The concentration of the culture supernatant is indicated relative to the concentration of a starting solution, which is designated 1, and the concentration of 0 represents a negative control subjected to heat treatment at 95°C for 30 minutes.

### Embodiments of the Invention

In one embodiment, the present disclosure provides a label capable of being adsorbed onto an electrode. This label can be bound to a substrate to form a substrate-label compound. In other words, in one embodiment, the present disclosure provides a substrate-label compound. The substrate-label compound may be a compound suitable for electrochemical measurement. That is, in one embodiment, the present disclosure provides a method of electrochemical measurement using a substrate-label compound. In another embodiment, the present disclosure provides a method of electrochemical measurement, said method comprising using a substrate-label compound labeled with a label, wherein the label is used to convert a substrate into a substrate-label compound suitable for electrochemical measurement by being bound to the substrate and wherein the label is capable of being adsorbed onto an electrode.

When the bond of the substrate-label compound is cleaved by the action of, e.g., a hydrolase, the label can be generated (produced). The generated label can be measured electrochemically. That is, in one embodiment, the present disclosure provides a method of electrochemical measurement, wherein a label is generated from a substrate-label compound by the action of a hydrolase and the generated label is measured electrochemically. As a substrate within the substrate-label compound, any compound selected to allow a hydrolase to act on the substrate-label compound can be used, and examples thereof include, but are not limited to, an amino acid, a peptide, a sugar, and a nucleic acid and the like.

The method of electrochemical measurement according to an embodiment of the present disclosure enables measurement of hydrolase activity of a specimen or a substance that activates a hydrolase, which may be contained in a specimen. Examples of a substance that activates a hydrolase can include endotoxin and (1→3)-β-D-glucan.

In an embodiment, various enzymes classified into Enzyme Class 3 can be used as a hydrolase. Examples thereof include, but are not limited to, proteases such as Factor C, Factor B, Factor G, and Proclotting enzyme (also referred to as a "clotting enzyme precursor") of horseshoe crab, subtilisin, proteinase K, trypsin, chymotrypsin, thrombin, plasmin, hemostatic factor Xa, hemostatic factor VIIa, hemostatic factor IXa, hemostatic factor XIa, hemostatic factor XIIa, protein C, kallikrein, papain, actinidine, caspase, pepsin, renin, chymosin, coagulase, calpain, angiotensin converting enzyme, and gingipain; esterases such as carboxyl esterase, triacylglycerol lipase, acetylcholine esterase, acetyl CoA hydrolase, phosphatases, such as alkaline phosphatase, acidic phosphatase, and protein phosphatase, 5'-nucleotidase and 3'-nucleotidase, glycosylases such as α-amylase and α-glucosidase, ether hydrolases, such as adenosyl homocysteinase, and alkenylglycerophosphocholine esterase, enzymes, such as asparaginase and urease, which act on a carbon-nitrogen bond other than a peptide bond, and enzymes, such as ATPase, which acts on an acid anhydride.

In one embodiment, the label can be a phenylenediamine-type compound. In one embodiment, the phenylenediamine-type compound may be a compound represented by Formula I below: 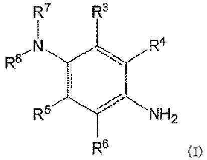 wherein
R⁷ represents hydrogen or linear or branched C₁₋₆ alkyl, C₁₋₆ alkenyl, C₁₋₆ alkynyl, C₃₋₉ cycloalkyl, phenyl, 1-naphthyl, 2-naphthyl, anthracenyl, or phenanthrenyl, which may be optionally substituted with one or more X;
R³, R⁴, R⁵, and R⁶ each independently represent hydrogen or linear or branched C₁₋₆ alkyl, C₁₋₆ alkenyl, C₁₋₆ alkynyl, C₁₋₆ alkoxy, halo, nitro, cyano, carboxy, sulfone, or amino, which may be optionally substituted with one or more Y; and
R⁸ is selected from the group consisting of phenyl, 1-naphthyl, 2-naphthyl, anthracenyl, and phenanthrennyl, which may be optionally substituted with one or more X,

wherein X represents linear or branched C₁₋₆ alkyl, C₁₋₆ alkenyl, C₁₋₆ alkynyl, C₁₋₆ alkoxy, anilino (i.e., PhNH-), phenoxy, halo, hydroxy, nitro, carboxy, cyano, sulfone, or amino, which may be optionally substituted with a substituent selected from the group consisting of halo, amino, cyano, carboxy, carbonyl, alkoxy, and sulfone, and
Y is selected from the group consisting of halo, amino, cyano, carboxy, carbonyl, alkoxy, and sulfone.

In one embodiment, the phenylenediamine-type compound may be N-(3-isopropylphenyl)-p-phenylenediamine.

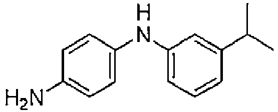

In one embodiment, the phenylenediamine-type compound may be N-(3-tert-butylphenyl)-p-phenylenediamine.

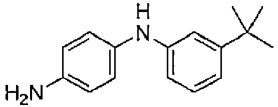

In one embodiment, the phenylenediamine-type compound may be N-(3,5-di-tert-butylphenyl)-p-phenylenediamine.

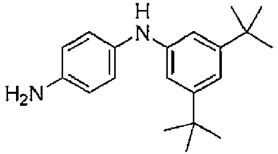

In one embodiment, the phenylenediamine-type compound may be N-(4-aminophenyl)-N' -phenyl-p-phenylenediamine.

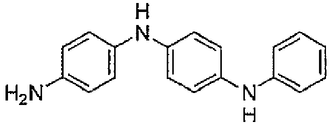

A phenylenediamine-type compound may be in a redox state or an ionized state. In the chemical formulae above, the phenylenediamine-type compound is described in a neutral and reduced form. However, the phenylenediamine-type compound is not limited to this form and may be in a reduced (diamine) form. When the released phenylenediamine-type compound adsorbs to the electrode and an electric potential is applied to the electrode, the phenylenediamine-type compound is converted into an oxidized (diimine) form.

In another embodiment, the label may be an aminoanthraquinone-based compound. In one embodiment, the aminoanthraquinone-based compound may be a compound represented by Formula II.

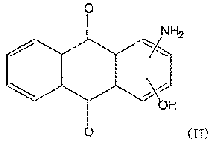

In one embodiment, the aminoanthraquinone-based compound may be 1-amino-4-hydroxyanthraquinone. 1-Amino-4-hydroxyanthraquinone has the structure shown below (CAS Number: 116-85-8).

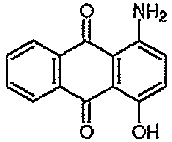

In another embodiment, the aminoanthraquinone-based compound may be 2-amino-3-hydroxyanthraquinone. 2-Amino-3-hydroxyanthraquinone has the structure shown below (CAS Number: 117-77-1). 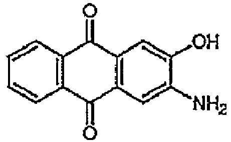 An aminoanthraquinone-based compound can be a salt, an acid addition salt, an anhydride, or a solvate thereof.

The term "alkyl" used herein refers to a linear or branched hydrocarbon having, for example, 6 carbon atoms. Examples of alkyl include, but are not limited to, methyl, ethyl, propyl, isopropyl, isobutyl, n-butyl, tert-butyl, isopentyl, n-pentyl, and heptyl.

The number of atoms (e.g., carbon atoms) used in the present specification is indicated as, for example, "Cx-y alkyl," which refers to an alkyl group having x to y carbon atoms. Other substituents and ranges are indicated in the same manner.

In the present specification, the phrase "which may optionally be substituted" or "substituted or unsubstituted" means an arbitrary substitution with one or more substituents and also includes a plurality of degrees of substitutions.

The aminoanthraquinone-based compound may be in a redox state or an ionized state. In the chemical formulae above, the aminoanthraquinone-based compound is described in a neutral and reduced form. However, the aminoanthraquinone-based compound is not limited to this form and may be in an oxidized form, a semi-oxidized form, or a reduced form. Further, the aminoanthraquinone-based compound may be in a neutral form or a cationic form. For the purpose of illustration, the term "an aminoanthraquinone-based compound," such as "an aminoanthraquinone-based compound represented by any of the chemical formulae," used herein refers to an aminoanthraquinone-based compound in a the neutral or cationic form, oxidized form, semi-oxidized form, or reduced form. For example, a neutral oxidized compound may be added as the aminoanthraquinone-based compound to a measurement system and then be converted to an oxidized cationic compound depending on the pH of the solution or due to electron transfer, and such compound is also within the scope of the aminoanthraquinone-based compound. Further, the term aminoanthraquinone-based compound also encompasses a salt, an acid addition salt, an anhydride, and a solvate thereof. Examples of the salt include, but are not limited to, salts of group 1 elements, salts of group 17 elements, such as Na salt, K salt, Cl salt, and Br salt and the like. Examples of the acid addition salt include, but are not limited to, hydrochloride, sulfate, sulfite, and nitrate.

The phenylenediamine-type compound and the aminoanthraquinone-based compound may be artificially synthesized or may be obtained as naturally occurring products. Further, commercially available products may be used. In the case of synthesizing the phenylenediamine-type compound and the aminoanthraquinone-based compound, organic synthesis may be performed using a conventional techniques of organic synthesis, and the products can be verified by NMR, IR, mass spectrometry, and the like.

The phenylenediamine-type compound and the aminoanthraquinone-based compound may be brought into contact with an electrode, so that they can be adsorbed onto the surface of the electrode. In this case, no special procedure, such as electrode surface activation via acid treatment, is necessary to allow the compounds to be adsorbed onto the electrode. In one embodiment, the phrase "a compound (a label) is capable of being adsorbed onto an electrode" refers to such compound being capable of being physically adsorbed onto an electrode. As the electrode, an electrode made of a material such as carbon, gold, or platinum can be used. The present disclosure also includes an embodiment in which the compound is allowed to be adsorbed onto a primary material, such as a carbon powder or a carbon material, and then the primary material, such as a carbon powder or a carbon material, is immobilized onto an electrode. It should be noted that this explanation is intended to describe the property of the compound and is not intended to limit the method for use of the compound. That is, in one embodiment, the present invention provides a method of allowing an compound to be adsorbed onto a primary material, such as a carbon powder or a carbon material, and then fixing the primary material, such as a carbon powder or a carbon material, onto an electrode.

In one embodiment, the present disclosure provides a reagent for electrochemical measurement of hydrolase activity or a substance that activates a hydrolase, said reagent comprising a substrate-label compound. The label in the substrate-label compound is capable of being adsorbed onto an electrode. In one embodiment, compounds that are not adsorbed onto the electrode are excluded from the label of the present disclosure.

Whether or not a label by itself or a label in the substrate-label compound is adsorbed onto an electrode can be confirmed by any method of electrochemical measurement described below. For example, whether or not a test compound is adsorbed onto an electrode can be confirmed by cyclic voltammetry. How the maximum value of oxidation current (IOmax) changes can be examined at varying scan rates in varying ranges, for example, from 4 mV/sec to 200 mV/sec. In general, when a label is adsorbed onto an electrode, it is known that in cyclic voltammetry the scan rate and the value of IOmax have a proportional relationship. Further, when the label is diffused without being adsorbed onto an electrode, IOmax is known to be proportional to one-half power of the scan rate (see, for example, "Electrochemical Measurement Manual Basics," edited by the Electrochemical Society of Japan, pp. 74-94). Based on such relationship between IOmax and the scan rate, it can be determined whether the compound is adsorbed onto an electrode or diffused without being adsorbed onto an electrode.

In one embodiment, the present disclosure provides a composition comprising the reagent for electrochemical measurement, which is used for measuring hydrolase activity or a substance that activates a hydrolase. In another embodiment, the present disclosure provides an electrode comprising the reagent or the composition for electrochemical measurement. In another embodiment, the present disclosure provides a system for electrochemical measurement involving the use of the electrode.

In one embodiment, the present disclosure provides a method for electrochemical measurement of a substance that activates a hydrolase, for example, a method for electrochemical measurement of a substance from a microbe such as endotoxin or (1→3)-β-D-glucan. Hereafter, a method for electrochemical measurement of endotoxin is described in detail as an example of the present disclosure.

When a specimen, which may contain endotoxin, is mixed with an LAL reagent, Factor C in the LAL reagent is activated by endotoxin, then Factor B is activated, and then Proclotting enzyme is activated into clotting enzyme. When clotting enzyme acts on the substrate-label compound of the present disclosure, a label is generated. A unit of endotoxin activity is indicated as "EU (endotoxin unit)." Unless specified otherwise, 1 EU corresponds to 1 international unit of endotoxin activity (IU) herein.

In one embodiment, the present disclosure provides a method for electrochemical measurement of trypsin. When trypsin acts on the substrate-label compound of the present disclosure, a label is generated. As the unit of trypsin activity, the amount of enzyme required to increase the absorption at 253 nm by 0.003 per minute at pH 7.6 and 25°C with N-benzoyl-L-arginine ethyl ester as the substrate is defined as 1 unit of enzyme activity (U). In another embodiment, the present disclosure provides a method for electrochemical measurement of gingipain. When gingipain acts on the substrate-label compound of the present disclosure, a label is generated. As the unit of gingipain activity, the amount of enzyme required to release 1 µmol of pNA in 1 hour at pH 7.6 and 37°C with N-benzoyl-L-arginine-p-nitroaniline as the substrate is defined as 1 unit of enzyme activity (U).

The generated label goes through an oxidation reaction at a particular potential and, therefore, it can be measured by an electrochemical method. In addition, the label of the present disclosure is capable of being adsorbed onto an electrode. Therefore, the label of the present disclosure is highly probable to be present in the vicinity of or on the surface of the electrode and, therefore, when compared with a label that is not capable of being adsorbed onto an electrode, use of the label of the present disclosure enables measurement with higher sensitivity. There is a correlation between the current value caused by the oxidation reaction of the label and the amount of the label generated; i.e., the endotoxin concentration. By utilizing such correlation, the concentration of the label can be quantified. In one embodiment, the label per se is oxidized while transferring an electron to the electrode without receiving an electron from another compound such as an oxidoreductase or coenzyme. In this embodiment, the label functions as a compound that releases an electron by itself instead of functioning as a mediator that merely mediates electron transfer from another compound such as an oxidoreductase or coenzyme, to the electrode.

In the measurement system, in addition to the label that is generated, a substrate-label compound that remains uncleaved may also be present in addition to the label generated. However, the redox potential of the label generated is different from the redox potential of the substrate-label compound and, therefore, the current due to by the label can readily be distinguished from the current due to by the substrate-label compound. Further, according to the method of the present disclosure, even a very small amount of label can be measured electrochemically and, therefore, the endotoxin concentration can be detected with high accuracy. Further, trypsin, gingipain, and phosphatase can be detected with high accuracy.

In a method of the present disclosure, the target substance is detected via electrochemical measurement, and the label is capable of being adsorbed onto an electrode and, therefore, a reaction on the electrode surface can be detected with high sensitivity, and the concentration of a substance can be measured even with a very small amount of specimen. Further, a trace substance contained in a specimen can be measured. In the method of the present disclosure, detection can be performed with high sensitivity and this means that the amount of a specimen necessary for detection of the same amount of a trace substance may be small. This also means that the amount of LAL reagent to be used can be reduced. Further, this means a trace substance can be measured more cost-effectively.

The present disclosure uses a substrate-label compound and utilizes the generated label. Because the label is stable in terms of electrochemical activity, accurate and stable detection is possible. Such properties are advantageous in, for example, long-term measurements. Further, in the method of the present disclosure, detection is not performed optically, unlike colorimetry or turbidimetry and, therefore, even a specimen with low transparency or a multicomponent specimen, such as tissue fluid, can be the subject of the measurement.

Examples of trace substances that can be measured by the method of the present disclosure include, but are not limited to, endotoxin, (1→3)-β-D-glucan, trypsin, gingipain, and phosphatase. In one embodiment, the trace substance to be measured is endotoxin. In another embodiment, the trace substance to be measured is (1→3)-β-D-glucan. In another embodiment, the trace substance to be measured is trypsin. In another embodiment, the trace substance to be measured is gingipain. In another embodiment, the trace substance to be measured is phosphatase. In one embodiment, the trace substance to be measured can be a substance from a microbe.

Here, a method for detection of endotoxin and/or (1→3)-β-D-glucan according to the present disclosure is described.

Reaction steps comprise bringing a specimen, which may contain endotoxin and/or (1→3)-β-D-glucan, into contact with an LAL reagent and a substrate-label compound. As a result, a label is generated from the substrate-label compound through a multi-stage reaction.

As the LAL reagent, LAL equivalents prepared from Factor C, Factor B, Factor G, and clotting enzyme and the like isolated and purified from organism-derived components or limulus factors, such as recombinant Factor C prepared via genetic engineering, may be used. Any species of horseshoe crabs can be used. Examples of horseshoe crabs include, but are not limited to, Tachypleus tridentatus, Limulus polyphemus, Carcinoscorpius rotundicauda, and Tachypleus gigas.

Commercially available LAL reagents can be used as the LAL reagent. Examples of commercially available reagents include the Kinetic-QCL (Lonza Walkersville Inc.), Endospecy (Seikagaku Corporation), Pyrochrome (Associates of Cape Cod, Inc.), Pyrotell-T (Associates of Cape Cod, Inc.), Pyrotell Multi-Test (Associates of Cape Cod, Inc.), and Endochrome-K (Charles River Laboratories).

The LAL reagent can be a reagent that is reconstituted to selectively comprise only particular limulus factors. In one embodiment, a reconstituted LAL reagent comprises Factor C and the reagent may or may not comprise other limulus factors (Factor B, Factor G, and Proclotting enzyme). In another embodiment, a reconstituted LAL reagent comprises Factor G and the reagent may or may not comprise other limulus factors (Factor C, Factor B, and Proclotting enzyme). A reconstituted LAL reagent can be prepared in accordance with conventional techniques (see, for example, Nakamura T., et al, J. Biochem., Mar 1986; 99 (3): 847-57). A recombinant limulus factor can be obtained by using a nucleic acid encoding a limulus factor gene to transform a host cell and expressing the gene therein. Examples of host cells include mammalian animal cells and insect cells and specific examples include, but are not limited to, Chinese hamster ovary-derived cells (CHO cells), human embryonic kidney-derived cells (HEK cells, such as HEK293 cells), and Sf9 cells (see, for example, WO 2012/118226 and WO 2014/092079).

In one embodiment, the substrate in the substrate-label compound may be an amino acid or oligopeptide. In such a case, the substrate-label compound can be an oligopeptide comprising a label bound to one terminus and an amino acid or peptide protective group bound to the other terminus. Such amino acid or oligopeptide can be, for example, an amino acid or oligopeptide indicated as an X-A-label, wherein X represents a protective group and A represents an amino acid or oligopeptide. Examples of the protective group X include, but are not limited to, groups that protect a peptide, such as a tert-butoxycarbonyl (Boc) group, a benzoyl group, a benzyl group, an acetyl (Ac) group, and a benzyloxycarbonyl (Cbz) group. A protective group may or may not be bound to an oligopeptide. Hereafter, a compound referred to as a substrate may or may not comprise a protective group bound thereto.

Examples of the amino acid include α-amino acids and β-amino acids, and the amino acid is not particularly limited, provided that it can release a label by the action of a lysate reagent. The oligopeptide is not particularly limited, provided that it can generate a label by the action of an LAL reagent. The oligopeptide can comprise, for example, 2 to 10, 2 to 5, or 3 or 4 amino acids, although the number of amino acids is not limited thereto. In one embodiment, the oligopeptide may comprise Arg (R) at its C terminus. Examples of oligopeptide include, but are not limited to, X-Asp-Pro-Arg (X-DPR), X-Val-Pro-Arg (X-VPR), X-Leu-Thr-Arg (X-LTR), X-Met-Thr-Arg (X-MTR), X-Leu-Gly-Arg (X-LGR), X-Thr-Gly-Arg (X-TGR), X-Ile-Glu-Gly-Arg (X-IEGR), X-Ser-Gly-Arg (X-SGR), X-Leu-Ala-Arg (X-LAR), X-Leu-Ser-Arg (X-LSR), X-Lys-Gly-Arg (X-KGR), X-Glu-Gly-Arg (X-EGR), X-Leu-Leu-Gly-Arg (X-LLGR), and X-Met-Leu-Gly-Arg (X-MLGR). In one embodiment, X can represent a peptide comprising 0 to 7 amino acids.

Example of the tripeptide include Leu-Gly-Arg, Thr-Gly-Arg, and Val-Pro-Arg. A specific example is a tripeptide comprising an L-amino acid represented by the general formula: R1-Gly-Arg-label, wherein R1 represents an N-blocked amino acid.

Another example is a tetrapeptide represented by the general formula: R2-A1-A2-A3-A4-label. In the formula, R2 represents hydrogen or a blocked aromatic hydrocarbon or acyl group; A1 represents an L-amino acid or D-amino acid selected from among Ile, Val, and Leu; A2 represents Glu or Asp; A3 represents Ala or Cys; and A4 represents Arg.

Specific examples of an oligopeptide comprising a label bound to one terminus and a peptide protective group bound to the other terminus include a Boc-Leu-Gly-Arg-label, an acetate-Leu-Gly-Arg-label, an acetate-Ile-Glu-Ala-Arg-label, a Boc-Val-Pro-Arg-label, and an acetate-Val-Pro-Arg-label.

In another embodiment, the substrate in the substrate-label compound may be a phosphoric acid group. In such a case, the substrate-label compound may be a phosphoric ester compound comprising a label bound to one terminus and a phosphoric acid group bound to the other terminus. Examples of structures of compounds are shown below.

In one embodiment, the substrate-label compound may be N-(3-isopropylphenyl)-p-phenylenediamine phosphate.

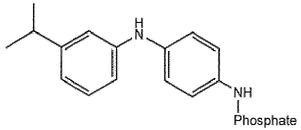

In one embodiment, the substrate-label compound may be N-(3-tert-butylphenyl)-p-phenylenediamine phosphate.

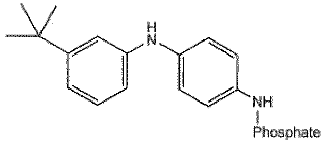

In one embodiment, the substrate-label compound may be N-(3,5-di-tert-butylphenyl)-p-phenylenediamine phosphate.

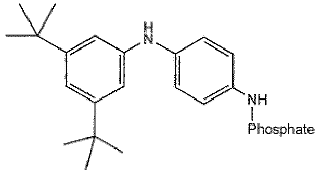

In one embodiment, the substrate-label compound may be N-(4-aminophenyl)-N'-phenyl-p-phenylenediamine phosphate.

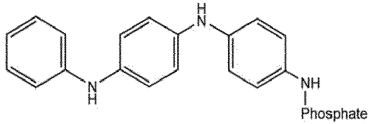

When bringing a specimen containing a trace substance into contact with an LAL reagent and a substrate-label compound, a buffer can be used. The buffer can be selected according to the type of hydrolase used in the measurement. For example, when an LAL reagent is used, a buffer having a pH of 6.0 to 9.0, such as a pH of 7.0 to 8.5, can be used. Examples of the buffer include, but are not limited to, Tris-acetate buffer, Tris-HCl buffer, phosphate buffer, HEPES buffer, and PIPES buffer. Likewise, when measuring other hydrolases such as trypsin, gingipain, and phosphatase (including alkaline phosphatase, acidic phosphatase, and protein phosphatase), a solution pH solution suitable for the enzyme reaction (e.g., pH 2 to 13) and a buffer suitable therefor can be selected.

In measurements using an LAL reagent, the label is released from the peptide substrate-label compound by an active clotting enzyme caused as a result of a multi-stage reaction. When carrying out the multi-stage reaction and the release reaction, the solution can be heated to activate the reaction. In the multi-stage reaction and the release reaction, the reaction temperature can be 20°C to 50°C, 25°C to 45°C, or 30°C to 40°C, for example, about 37°C. In one embodiment, the reaction duration can be, for example, 1 minute or longer, 5 minutes or longer, 10 minutes or longer, 15 minutes or longer, 20 minutes or longer, 30 minutes or longer, 1 hour or longer, or 2 hours or longer, although the reaction duration is not limited thereto. In another embodiment, the reaction duration can be, for example, 2 hours or shorter, 1 hour or shorter, 30 minutes or shorter, 20 minutes or shorter, 15 minutes or shorter, 5 minutes or shorter, or 1 minute or shorter, although the reaction duration is not limited thereto.

When measuring other hydrolases such as trypsin, gingipain, and phosphatase (including alkaline phosphatase, acidic phosphatase, and protein phosphatase), a label is released from the substrate-label compound by the action of a hydrolase. In order to activate the hydrolysis reaction, the solution can be heated. The reaction temperature can be 20°C to 50°C, 25°C to 45°C, or 30°C to 40°C, for example, about 37°C. In one embodiment, the reaction duration can be, for example, 1 minute or longer, 5 minutes or longer, 10 minutes or longer, 15 minutes or longer, 20 minutes or longer, 30 minutes or longer, 1 hour or longer, or 2 hours or longer, although the reaction duration is not limited thereto. In another embodiment, the reaction duration can be, for example, 2 hours or shorter, 1 hour or shorter, 30 minutes or shorter, 20 minutes or shorter, 15 minutes or shorter, 10 minutes or shorter, 5 minutes or shorter, or 1 minute or shorter, although the reaction duration is not limited thereto.

In one embodiment, endotoxin at a concentration of, for example, 0.1 EU/l or higher, 0.2 EU/l or higher, 0.3 EU/l or higher, 0.4 EU/l or higher, 0.5 EU/l or higher, 0.6 EU/l or higher, 0.7 EU/l or higher, 0.8 EU/l or higher, 0.9 EU/l or higher, 1 EU/l or higher, 2 EU/l or higher, 3 EU/l or higher, 4 EU/l or higher, 5 EU/l or higher, 6 EU/l or higher, 7 EU/l or higher, 8 EU/l or higher, 9 EU/l or higher, or 10 EU/l or higher can be measured by the method of the present disclosure. In one embodiment, endotoxin at a concentration of, for example, 1000 EU/l or lower, 900 EU/l or lower, 800 EU/l or lower, 700 EU/l or lower, 600 EU/l or lower, 500EU/l or lower, 400 EU/l or lower, 300 EU/l or lower, 200 EU/l or lower, 100 EU/l or lower, 90 EU/l or lower, 80 EU/l or lower, 70 EU/l or lower, 60 EU/l or lower, 50 EU/l or lower, 40 EU/l or lower, 30 EU/l or lower, 20 EU/l or lower, or 10 EU/l or lower can be measured by the method of the present disclosure. In another embodiment, trypsin, gingipain, or phosphatase at a concentration of, for example, 0.1 U/l or higher, 0.2 U/l or higher, 0.5 U/l or higher, 1 U/l or higher, 1.5 U/l or higher, 2 U/l or higher, 5 U/l or higher, 10 U/l or higher, 50 U/l or higher, 100 U/l or higher, or 1000 U/l or higher can be measured by the method of the present disclosure. In another embodiment, trypsin, gingipain, or phosphatase at a concentration of, for example, 10000 U/l or lower, 5000 U/l or lower, 1000 U/l or lower, 500 U/l or lower, 100 U/l or lower, 50 U/l or lower, or 10 U/l or lower can be measured by the method of the present disclosure. Unless otherwise specified, a numerical range in the present disclosure includes the upper limit and the lower limit (e.g., a numerical range of "a" to "b" indicates a range of no less than "a" to no more than "b"). Also, the present disclosure encompasses any combinations of the upper limit and lower limit exemplified concerning the numerical range. In one embodiment, endotoxin at a concentration of 0.1 to 1000 EU/l, for example, 1 to 1000 EU/l can be measured by the method of the present disclosure. In another embodiment, trypsin, gingipain, or phosphatase at a concentration of 0.1 to 10000 U/l, for example, 1 to 1000 U/l can be measured by the method of the present disclosure.

In one embodiment, the present disclosure provides a reagent for electrochemical measurement of hydrolase activity or a substance that activates a hydrolase comprising a substrate-label compound or provides a composition comprising such reagent used for measurement of a substance. The reagent or composition may contain various substances that are necessary for measurement. The concentration of the substrate-label compound in the reagent or composition can be appropriately selected in accordance with measurement. For example, such concentration can be 0.01% (w/w) or higher, 0.02% (w/w) or higher, 0.03% (w/w) or higher, 0.04% (w/w) or higher, 0.05% (w/w) or higher, 0.1% (w/w) or higher, 0.2% (w/w) or higher, 0.3% (w/w) or higher, 0.4% (w/w) or higher, 0.5% (w/w) or higher, 1% (w/w) or higher, 2% (w/w) or higher, 3% (w/w) or higher, 4% (w/w) or higher, 5% (w/w) or higher, 10% (w/w) or higher, 20% (w/w) or higher, 30% (w/w) or higher, 40% (w/w) or higher, or 50% (w/w) or higher, although the concentration is not limited thereto. In another embodiment, the concentration of the substrate-label compound in the reagent or composition can be, for example, 99% (w/w) or lower, 90% (w/w) or lower, 50% (w/w) or lower, 40% (w/w) or lower, 30% (w/w) or lower, 20% (w/w) or lower, 10% (w/w) or lower, 5% (w/w) or lower, 4% (w/w) or lower, 3% (w/w) or lower, 2% (w/w) or lower, 1% (w/w) or lower, 0.5% (w/w) or lower, 0.4% (w/w) or lower, 0.3% (w/w) or lower, 0.2% (w/w) or lower, or 0.1% (w/w) or lower, although the concentration is not limited thereto. The percentage by weight as used herein is the percentage by weight based on dry weight.

The measurement step comprises subjecting the solution after the release reaction described above to an electrochemical reaction. Thus, a trace substance (e.g., a substance from a microbe) can be quantified based on the measured current value. In the solution after the reaction, the label generated from the substrate-label compound is present, and the label is oxidized at a particular potential. A correlation holds between the current value due to the oxidation reaction and the amount of the label generated; i.e., the concentration of a trace substance (e.g., a substance from a microbe). By utilizing such correlation, the concentration of the label can be quantified.

Measurement via an electrochemical reaction can be performed by any method. Examples of methods of measurement include, but are not limited to, the amperometry method and the voltammetry method and the like. Examples of amperometry methods include, but are not limited to, the chronoamperometry method and the differential pulse amperometry method and the like. Examples of voltammetry methods include, but are not limited to, the normal pulse voltammetry method, the differential pulse voltammetry method, and the cyclic voltammetry method and the like.

In the amperometry method, for example, an electrode is introduced into a mixture of a specimen, an LAL reagent, and a substrate-label and subjected to measurement by the amperometry method. In one embodiment, a given potential is applied to the working electrode and the generated current is measured in this state. The potential is regulated against the reference electrode. A current is generated between the working electrode and the counter electrode. When the current value is sufficiently low, the counter electrode may be omitted. In such a case, the reference electrode can serve as the counter electrode. The duration of voltage application may be plotted on the horizontal axis and the current value may be plotted on the vertical axis, so as to prepare a chart showing current values after the elapse of a given time after a given potential is applied to the electrode. A calibration curve showing the correlation between a specimen having a known concentration, such as a trace substance having a known concentration, and the current value after the elapse of a given time can be prepared in advance. Thus, the concentration of a trace substance contained in a test specimen, which may contain a substance from a microbe having an unknown concentration, can be calculated based on the measured current value. The voltage to be applied can be set to, for example, - 1000 mV to +1000 mV (vs. Ag/AgCl), although this may differ depending on conditions or apparatus settings.

The electrode is not limited and any common electrode used for electrochemical measurement can be used. For example, as the working electrode, a carbon electrode, such as a glassy carbon, carbon paste, graphite, or diamond-like carbon electrode, or an electrode made of electrochemically stable noble metals, such as gold or platinum, can be used. As the counter electrode, an electrode made of electrochemically stable noble metals, such as gold or platinum, can be used. As the reference electrode, for example, an Ag/AgCl electrode can be used.

As the measurement apparatus, an apparatus that is commonly used for electrochemical measurement can be used, and an example thereof is an apparatus equipped with a potentiostat, a current amplifier, and an information processing unit. The information processing unit can be equipped with a CPU, memory, or an external memory mechanism such as a HDD, a means of communication such as a modem, a display, and a means of input, such as a mouse and a keyboard. The information processing unit can analyze electric signals according to the programs set in given regions, such as the internal memory or external memory apparatus, and determine the concentration of a trace substance. The information processing apparatus may be a general-purpose or dedicated apparatus.

### Preparation of the substrate-label compound

The label of the present disclosure can be ligated to a substrate. In one embodiment, the substrate-label compound comprises a label bound to one of its termini and a peptide protective group bound to the other terminus. That is, the substrate-label compound can be a compound that is a protective group-substrate-label. In another embodiment, the substrate-label compound comprises a label bound to one of its termini and a phosphoric acid group bound to the other terminus. That is, the substrate-label compound can be a compound that is a phosphoric acid-label. In one embodiment, the substrate-label compound is a compound with no protective group bound thereto. The substrate to be used can be synthesized according to a known method of synthesis. The structure of the substrate can be designed according to the substrate specificity or reactivity of the hydrolase. The label can be bound to the substrate by a general method used in the field of organic synthesis. The prepared substrate-label compound can be separated and/or purified via thin-layer chromatography using silica gel columns or the like, and the product can be confirmed via mass analysis or NMR.

The present disclosure is implemented using conventional techniques of chemistry, organic synthesis, biochemistry, molecular biology, or electrochemistry, and such techniques are within the competence of a person skilled in the art. Such techniques are described in the literature. See, for example, Organic Chemistry (Jonathan Clayden (ed.), Nick Greeves, Stuart Warren, Peter Wothers), Oxford Univ. Pr, 2000, and March's Advanced Organic Chemistry: Reactions, Mechanisms, and Structure (Michael B. Smith, Jerry March), Wiley-Interscience, 6th edition, 2007. Each of these general textbooks are incorporated herein by reference.

As demonstrated in the examples, the method of the present disclosure enables measurement of endotoxin. Further, as demonstrated in the examples, the method of the present disclosure enables measurement of trypsin. Further, as demonstrated in the examples, the method of the present disclosure enables measurement of gingipain. Such methods are characterized in that a label capable of being adsorbed onto an electrode is used and the label is measured by electrochemical measurement. The method of the present disclosure does not depend on the particular type of hydrolase and, as long as a label is released, a target substance can be electrochemically measured based on the same principle.

### Examples

Hereafter, the present disclosure is further illustrated with reference to the examples described below. However, the technical scope of the present disclosure is not limited to these examples in any way. In the examples, the compounds and the like below may be abbreviated as follows.
N,N-diisopropylethylamine: N,N-DIPEA or simply DIPEA
Triethylamine: Et₃N
Dimethyl sulfoxide: DMSO
Methanol: MeOH
Ethyl acetate: AcOEt
Palladium carbon: Pd/C
Heating: Δ
Water-soluble carbodiimide hydrochloride (1-Ethyl-3-(3-dimethylaminopropyl)carbodiimide hydrochloride): WSCl·HCl or EDC·HCl
Dichloromethane: DCM or CH₂Cl₂
Trifluoroacetic acid: TFA
tert-Butoxycarbonyl group: Boc
Benzyloxycarbonyl group: Z or Cbz

### Material and method

Unless specified otherwise, materials and reagents were commercially available or were obtained or prepared according to routine approaches of the technical field or procedures of literatures known in the art. As the LAL reagent, the Endospecy ES-24S set (manufactured by Seikagaku Corporation) or Limulus Color KY Single Test Wako (Product Code: 291-53601, FUJIFILM Wako Pure Chemical Corp.) was used. The endotoxin standard solution was prepared by diluting an USP Reference Standard endotoxin (manufactured by Seikagaku Corporation) to a given concentration with endotoxin-free distilled water. Porcine pancreas-derived trypsin (Product Code: 201-19181, manufactured by FUJIFILM Wako Pure Chemical Corp.) was used as the trypsin. Alkaline phosphatase was available from, for example, FUJIFILM Wako Pure Chemical Corp. (alkaline phosphatase, Product Code: 018-10693. The compounds of N-(3-isopropylphenyl)p-phenylenediamine, N-(3-tert-butylphenyl)-p-phenylenediamine, N-(3,5-di-tert-butylphenyl)-p-phenylenediamine, and N-(4-aminophenyl)-N'-phenyl-p-phenylenediamine, and a substrate-peptide compound comprising primary amino groups of such 4 compounds amide-linked to the carboxyl group of the Arg residue of Leu-Gly-Arg or Val-Pro-Arg were obtained from Watanabe Chemical Industries, Ltd. Such compounds were prepared in the manner described below.

The following scheme was used to synthesize N-(3-isopropylphenyl)-p-phenylenediamine, N-(3-tert-butylphenyl)-p-phenylenediamine, N-(3,5-di-tert-butylphenyl)-p-phenylenediamine, and N-(4-aminophenyl)-N'-phenyl-p-phenylenediamine (indicated as KIK-MO-xx in the scheme).

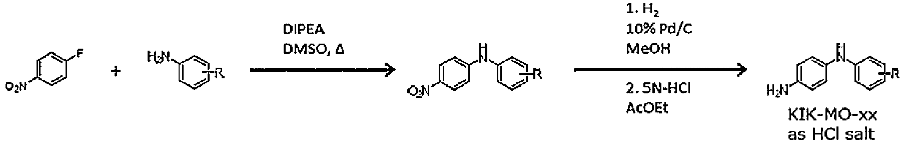

A reaction was allowed to proceed with the addition of a base to 4-fluoronitrobenzene and an arylamine corresponding to target compound (e.g., 3-isopropylaniline, 3-tert-butylaniline, or 3,5-di-tert-butylaniline) to synthesize a compound having a diphenylamine backbone, and a nitro group was then reduced to an amino group via catalytic reduction thereby resulting in synthesis of the target compound.

The following scheme was used to bind a substrate; i.e., Leu-Gly-Arg or Val-Pro-Arg, to the compounds of N-(3-isopropylphenyl)-p-phenylenediamine, N-(3-tert-butylphenyl)-p-phenylenediamine, N-(3,5-di-tert-butylphenyl)-p-phenylenediamine, and N-(4-aminophenyl)-N'-phenyl-p-phenylenediamine (indicated as KIK-PE-xx in the scheme). Further, in the scheme, the label compound is indicated generically as "NH₂-Ar."

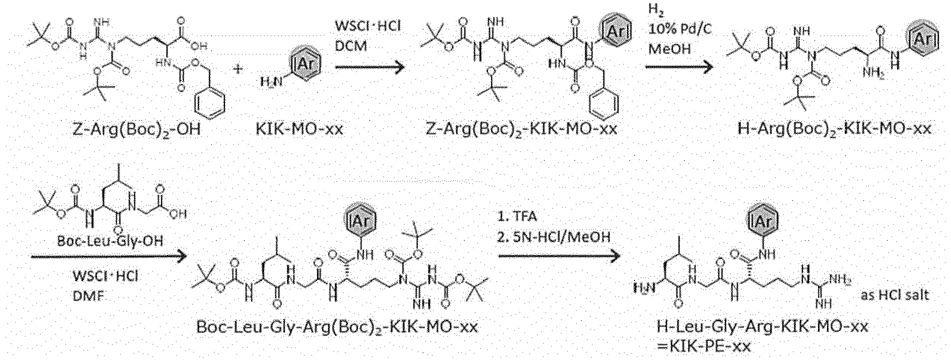

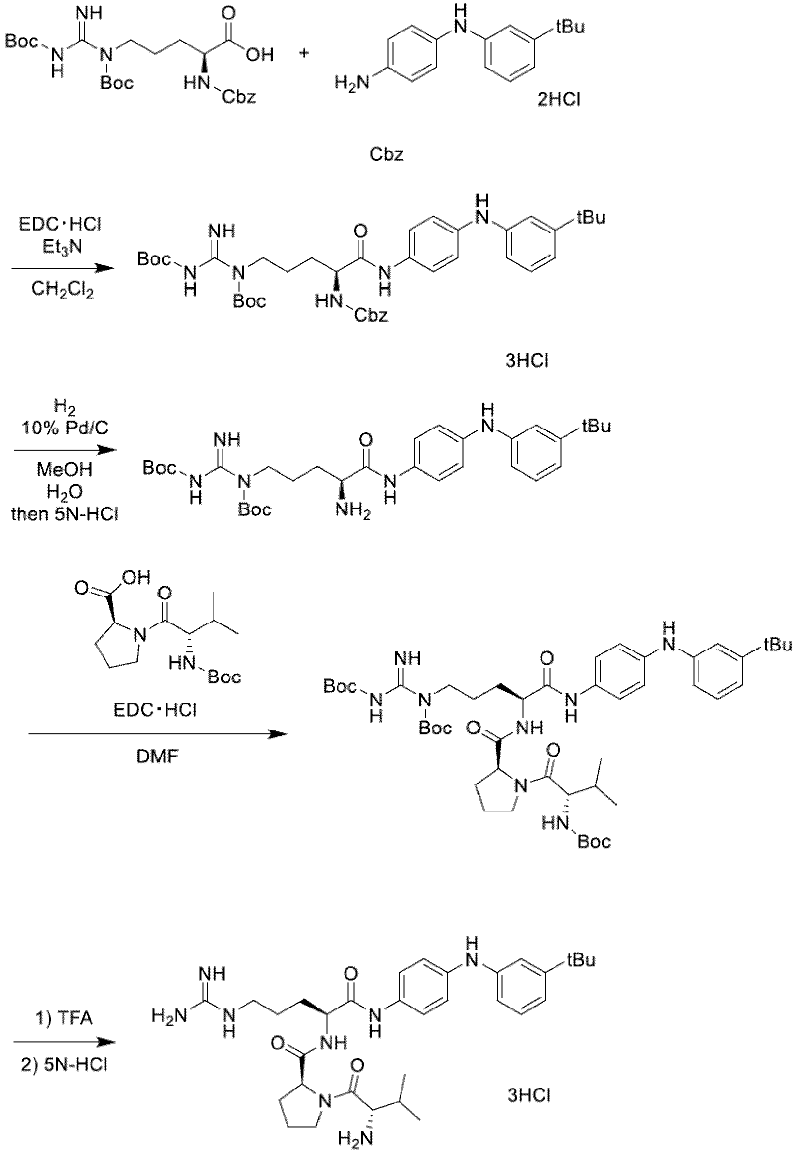

KIK-MO-xx synthesized in advance and an amino acid or oligopeptide were protected with a protective group, such as a Z or Boc group, and then bound to each other using a condensing agent, such as aqueous carbodiimide, thereby resulting in synthesis of the target compound.

Products were inspected via HPLC and LC/MS. Concerning other compounds, commercially available products were obtained from Tokyo Chemical Industry Co., Ltd., FUJIFILM Wako Pure Chemical Corp., and Nacalai tesque, INC.

### [Example 1] Confirmation of adsorbability of the phenylenediamine-type compound onto a carbon electrode

Cyclic voltammetry of a glassy carbon electrode was performed using 4 types of phenylenediamine-type compounds; i.e., N-(3-isopropylphenyl)-p-phenylenediamine, N-(3-tert-butylphenyl)-p-phenylenediamine, N-(3,5-di-tert-butylphenyl)-p-phenylenediamine, and N-(4-aminophenyl)-N'-phenyl-p-phenylenediamine. More specifically, 10 µl of a solution of a 1 mM phenylenediamine-type compound, 90 µl of 100 mM potassium phosphate buffer (pH 7.0), and 100 µl of ultrapure water were mixed to prepare a measurement solution. Cyclic voltammetry was performed using a glassy carbon rod electrode (an SGCE glassy carbon electrode, 3×1.6, BAS) as the working electrode, a platinum rod electrode (a PT counter electrode, 5.7 cm, BAS) as the counter electrode, and a silver/silver chloride electrode (RE-11A, EC Frontier Co., Ltd.) as the reference electrode. The scan range was from -400 mV to +800 mV. The scan rate was altered in a range of from 4 mV/sec to 200 mV/sec to examine changes in the maximal oxidation current (IOmax). When a compound is adsorbed onto an electrode, in general, it is known that a proportional relationship exists between the scan rate and the IOmax value in cyclic voltammetry. When a compound is diffused in a solution, IOmax is proportional to one-half power of the scan rate.

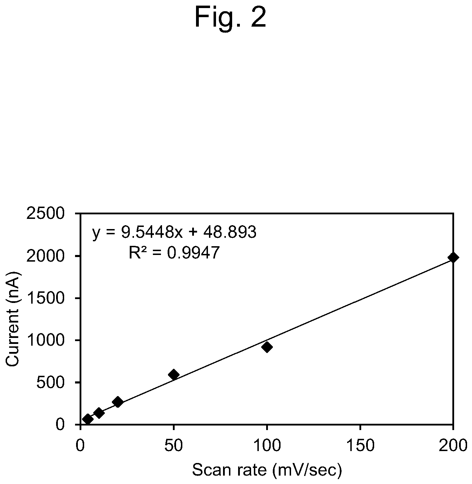

Figure 2 shows the results of performing cyclic voltammetry using N-(3-isopropylphenyl)p-phenylenediamine and plotting the scan rate and IOmax. The results demonstrate that a proportional relationship exists between the scan rate and the IOmax value and that N-(3-isopropylphenyl)-p-phenylenediamine is adsorbed onto a carbon electrode.

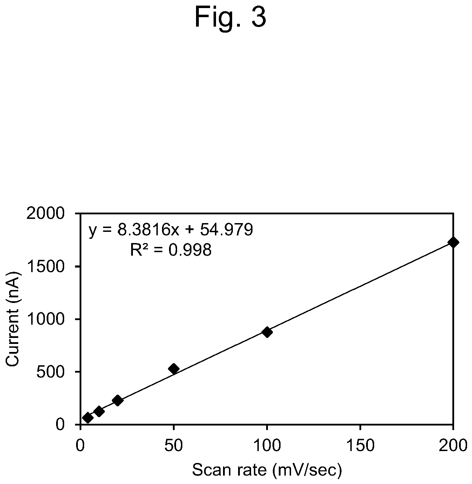

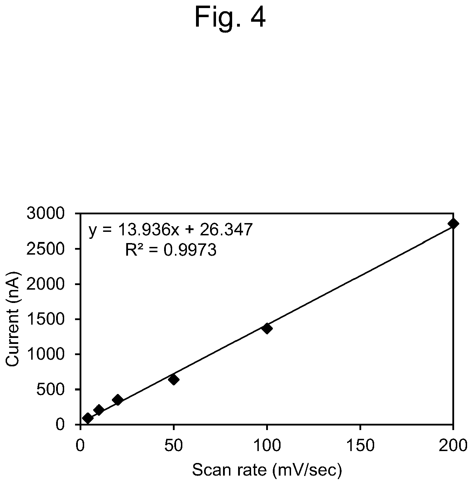

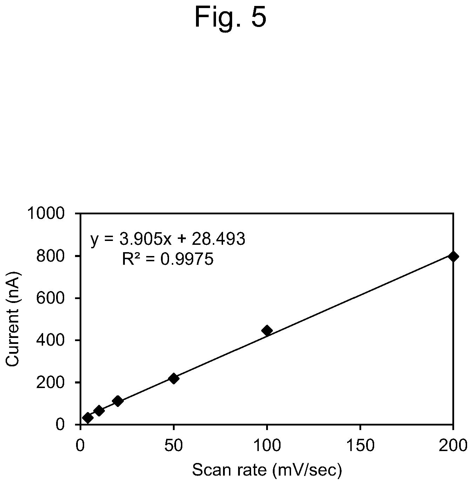

Figures 3, 4, and 5 each show the results of cyclic voltammetry performed in the same manner except for the use of N-(3-tert-butylphenyl)-p-phenylenediamine, N-(3,5-di-tert-butylphenyl)-p-phenylenediamine, and N-(4-aminophenyl)-N'-phenyl-p-phenylenediamine instead of N-(3-isopropylphenyl)p-phenylenediamine. The results demonstrate that a proportional relationship exists between the scan rate and the IOmax value in all compounds and that N-(3-isopropylphenyl)-p-phenylenediamine is adsorbed onto a carbon electrode as with carbon.

### Comparative Example (p-aminophenol)

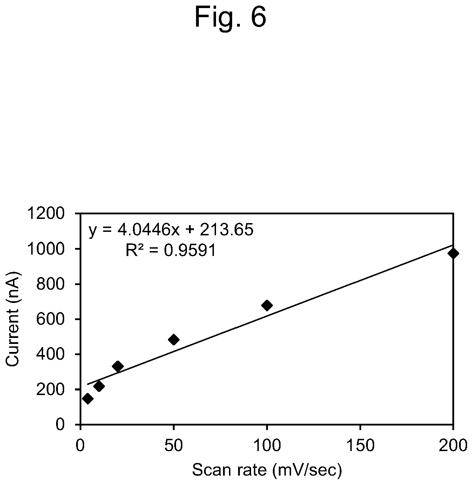

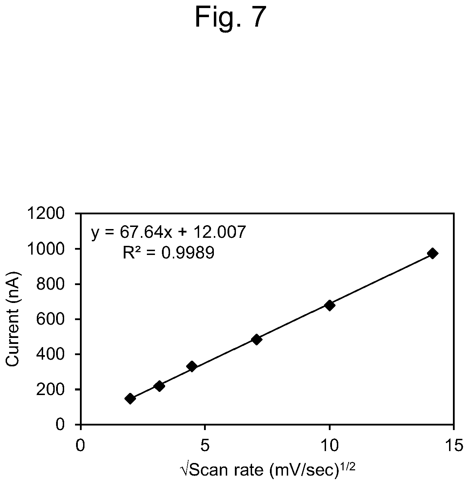

Figures 6 and 7 each show the results of cyclic voltammetry performed in the same manner except for the use of p-aminophenol, which is a known label, instead of the phenylenediamine-type compound of the present disclosure. The results demonstrate that IOmax is proportional to one-half power of the scan rate and that p-aminophenol is not adsorbed onto the electrode but diffused.

### [Example 2] Measurement of endotoxin using a substrate-label compound

A compound comprising Leu-Gly-Arg and N-(3-isopropylphenyl)-p-phenylenediamine bound to each other (hereafter referred to as "LGR-N-(3-isopropylphenyl)-p-phenylenediamine") was used to perform electrochemical measurement of endotoxin. More specifically, 200 µl of a buffer included in the Endospecy ES-24S set was added to and thoroughly mixed with the LAL reagent included in the Endospecy ES-24S set to prepare an LAL solution. In a dry-heat-sterilized test tube, 10 µl of a solution of 1 mM LGR-N-(3-isopropylphenyl)-p-phenylenediamine, 90 µl of the LAL solution, and 90 µl of an aqueous solution of endotoxin at a given concentration (0, 1, 10, 100, or 1000 EU/l) were mixed, and the resultant was allowed to stand in an incubator at 37°C for 1 hour. Thereafter, cyclic voltammetry was performed using the same electrode as used in Example 1 and the same scan range as in Example 1, except that the scan rate was set at 20 mV/sec.

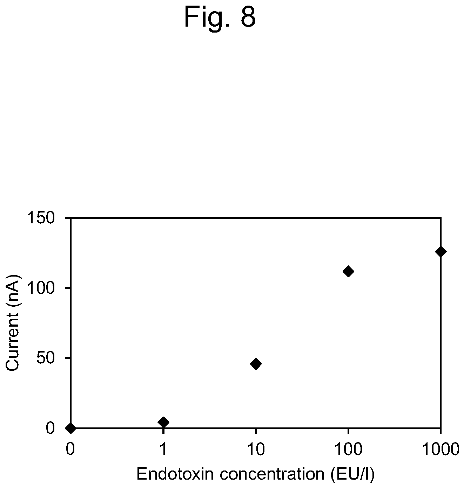

Figure 8 shows the results of plotting the endotoxin concentration on the horizontal axis and the oxidation current at 120 mV (vs.Ag/AgCl) on the vertical axis. The results demonstrate that the current value increases in an endotoxin concentration-dependent manner. 120 mV (vs.Ag/AgCl) is the oxidation potential of N-(3-isopropylphenyl)p-phenylenediamine, which is the label, and a correlation is observed between the endotoxin concentration and the label-derived oxidation current. This indicates that electrochemical measurement enables quantification of the endotoxin concentration in the specimen.

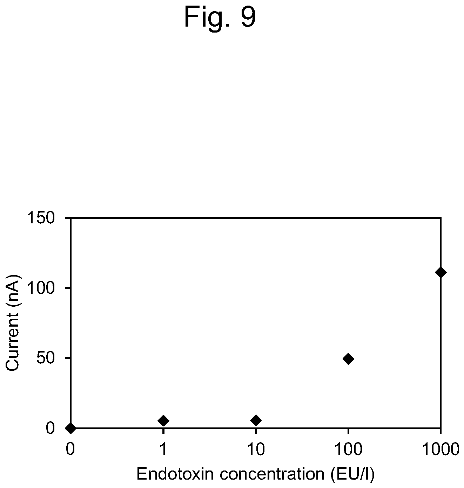

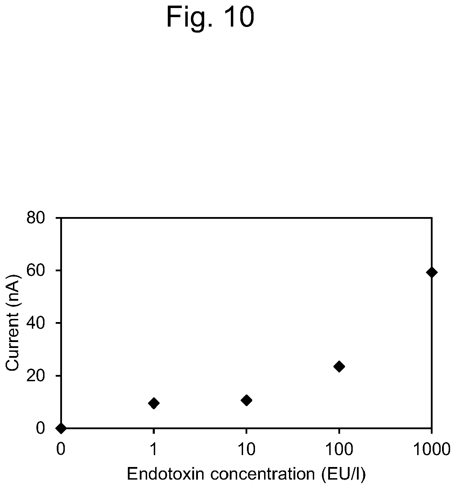

Compounds each comprising Leu-Gly-Arg and N-(3-tert-butylphenyl)-p-phenylenediamine or Leu-Gly-Arg and N-(3,5-di-tert-butylphenyl)-p-phenylenediamine bound thereto instead of LGR-N-(3-isopropylphenyl)p-phenylenediamine (hereafter indicated as LGR-N-(3-tert-butylphenyl)-p-phenylenediamine or LGR-N-(3,5-di-tert-butylphenyl)-p-phenylenediamine) were used to perform the same test. The results are shown in Figures 9 and 10. With regard to the oxidation current values shown on the vertical axis, the current of LGR-N-(3-tert-butylphenyl)-p-phenylenediamine was the current measured when applying 120 mV and the current of LGR-N-(3,5-di-tert-butylphenyl)-p-phenylenediamine was the current measured when applying 110 mV. The results demonstrate that the oxidation current value increases in an endotoxin concentration-dependent manner and that the endotoxin concentration can be measured by the method of the present disclosure.

### [Example 3] Measurement of low-concentration endotoxin using a substrate-label compound

LGR-N-(3-isopropylphenyl)-p-phenylenediamine was used to perform electrochemical measurement of low-concentration endotoxin. More specifically, 200 µl of endotoxin-free distilled water was added to and thoroughly mixed with the LAL reagent included in Limulus Color KY Single Test Wako to prepare an LAL solution. In a dry-heat-sterilized test tube, 90 µl of the LAL solution and 90 µl of an aqueous solution of endotoxin at a given concentration (0, 1, 5, or 10 EU/l) were mixed, and the resultant was allowed to stand in an incubator at 37°C for 1 hour. Thereafter, 10 µl of a solution of 4 mM LGR-N-(3-isopropyl)-p-phenylenediamine was added, and the resultant was allowed to stand in an incubator at 37°C for an additional 1 hour. After the completion of the reaction, chronoamperometry was performed using the same electrode as used in Example 1. A potential of 0 mV (vs. Ag/AgCl) was first applied for 10 seconds, and then a step potential of 250 mV (vs. Ag/AgCl) was applied. The current value measured 1 second after the initiation of applying the potential of 250 mV was designated as the measurement current value.

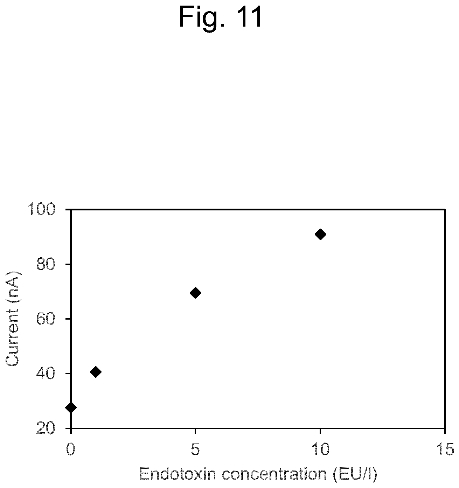

Figure 11 shows the results of plotting the endotoxin concentration on the horizontal axis and the measurement current value on the vertical axis. The results demonstrate that the current value increases in an endotoxin concentration-dependent manner. When a potential of 250 mV (vs.Ag/AgCl) is applied, the substrate-label; i.e., LGR-N-(3-isoporopyl)-p-phenylenediamine, is not oxidized, but only the label; i.e., N-(3-isopropylphenyl)p-phenylenediamine, is oxidized, thereby leading to a correlation between the endotoxin concentration and the measurement current value. This indicates that electrochemical measurement enables quantification of the endotoxin concentration in the specimen.

### [Example 4] Measurement of trypsin using a substrate-label compound

LGR-N-(3-tert-butylphenyl)-p-phenylenediamine was used to perform electrochemical measurement of trypsin. Trypsin was diluted to a given concentration using 0.1 mM Tris-HCl (pH 7.5). In a dry-heat-sterilized test tube, 10 µl of a solution of 1 mM LGR-N-(3-tert-butylphenyl)-p-phenylenediamine, 90 µl of an aqueous solution of trypsin at a given concentration (final concentration of 0, 630, or 6300 U/l), and 90 µl of ultrapure water were mixed, and the resultant was allowed to stand in an incubator at 37°C for 1 hour. Thereafter, cyclic voltammetry was performed under the same conditions as in Example 2.

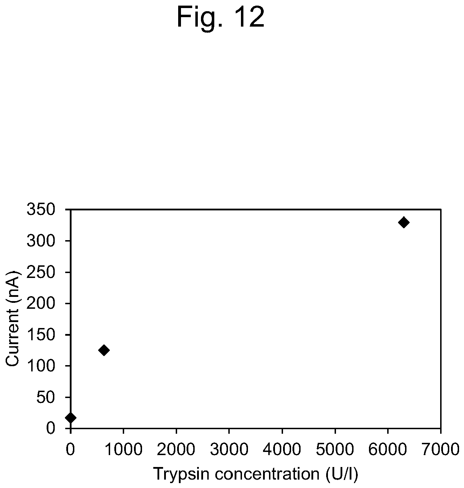

Figure 12 shows the results of plotting the trypsin concentration on the horizontal axis and the oxidation current at 120 mV (vs.Ag/AgCl) on the vertical axis. The results demonstrate that the current value increases in a trypsin-concentration-dependent manner. 120 mV (vs.Ag/AgCl) is the oxidation potential of N-(3-tert-butylphenyl)-p-phenylenediamine as the label, and a correlation is observed between the trypsin concentration and the label-derived oxidation current. This indicates that electrochemical measurement enables quantification of the trypsin concentration in the specimen.

### [Example 5] Measurement of gingipain using a substrate-label compound

*P. gingivalis* JCM 8525 cells used in the measurement were cultured in the manner described below. A 5 mg/ml hemin stock solution was prepared by dissolving 0.05 mg of hemin (H0008, Tokyo Chemical Industry Co., Ltd.) with the addition of 1 ml of 1 N aqueous ammonia, adding 9 ml of distilled water, and heat-sterilizing the mixture in an autoclave. A 1 mg/ml vitamin K stock solution was prepared by dissolving 0.1 g of 2-methyl-1,4-naphthoquinone (M0373, Tokyo Chemical Industry Co., Ltd.) with the addition of 100 ml of ethanol (Wako Pure Chemical Corp.). These solutions were refrigerated after preparation. A BHI agar medium was prepared by dissolving 5.2 g of the pearl core brain heart infusion (BHI) agar medium "Eiken" (E-MC61, Eiken Chemical Co., Ltd.) in distilled water, adding 0.1 ml of the hemin stock solution (final concentration of 5 µg/ml) and the vitamin K stock solution (final concentration of 1 µg/ml) to adjust the volume of the solution to 100 ml, and sterilizing the resultant in an autoclave. The sterilized agar medium while still hot was spread onto a sterilized petri dish in an amount of approximately 20 ml per dish, cooled to solidify, stored in a hermetically sealed container containing the AnaeroPack (Mitsubishi Gas Chemical Company, Inc.) therein, and degassed. The BHI liquid medium was prepared by adding 0.1 ml of the hemin stock solution (final concentration of 5 µg/ml) and the vitamin K stock solution (final concentration of 1 µg/ml) to 3.7 g of the pearl core brain heart infusion broth medium "Eiken" (E-MC62, Eiken Chemical Co., Ltd.) to adjust the volume of the solution to 100 ml, aliquoting the resultant in an amount of 2 ml each to silicon-capped test tubes, and sterilizing the resultant in an autoclave. After cooling, the resultant was stored in a hermetically sealed container containing the AnaeroPack (Mitsubishi Gas Chemical Company, Inc.) therein and then degassed. The cells were seeded from the glycerol stock to the BHI agar medium using an inoculation loop. Culture was performed with degassing in a hermetically sealed container containing the AnaeroPack (Mitsubishi Gas Chemical Company, Inc.) therein via static culture at 37°C for approximately 1 week. When colonies were detected, the culture product was subcultured to the BHI liquid medium, and culture was then performed with degassing in a hermetically sealed container containing the AnaeroPack (Mitsubishi Gas Chemical Company, Inc.) therein via static culture at 37°C for approximately 1 week. The culture was completed when a sufficient level of turbidity was observed. The culture solution was centrifuged at 4300× g for 20 minutes, and cells were removed from the supernatant using a 0.22-µm filter to obtain a stock solution of the culture supernatant. The stock solution of the culture supernatant was adequately diluted using phosphate buffered physiological saline (pH 7.4) (Product Code: 27575-31, Nacalai tesque, INC.).

A compound of Val-Pro-Arg bound to N-(3-tert-butylphenyl)-p-phenylenediamine (hereafter indicated as "VPR-N-(3-tert-butylphenyl)-p-phenylenediamine") was used to perform electrochemical measurement of gingipain. More specifically, 10 µl of a solution of 1 mM VPR-N-(3-tert-butylphenyl)-p-phenylenediamine, 10 µl of a culture supernatant solution of *P. gingivalis* at a given concentration (a stock solution, a 2-fold diluted solution, or a 4-fold diluted solution), and 80 µl of phosphate buffered physiological saline (pH 7.4) were mixed in a dry-heat-sterilized test tube, and the resultant was allowed to stand in an incubator at 37°C for 1 hour. Thereafter, cyclic voltammetry was performed under the same conditions as in Example 2. As a negative control, a stock solution of the culture supernatant of *P. gingivalis* was heat-treated at 95°C for 30 minutes, and measurement was performed in the same manner using the resulting solution.

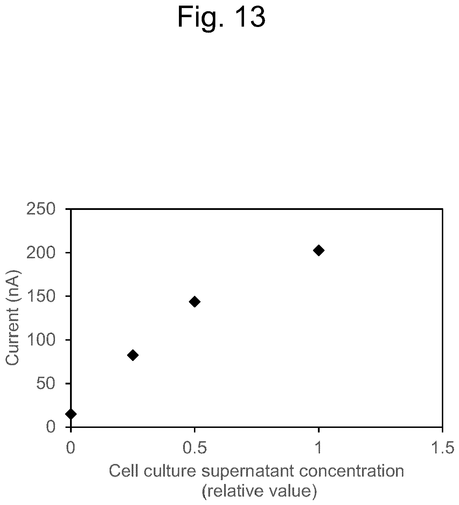

Figure 13 shows the results of plotting the concentration of the assay sample relative to the concentration of the stock solution of the culture supernatant, which is designated to be 1, on the horizontal axis and the oxidation current at 120 mV (vs.Ag/AgCl) on the vertical axis. The relative concentration 0 indicates a negative control prepared by heat treatment of the stock solution of the culture supernatant. The results demonstrate that a current value increases in a culture-supernatant-concentration-dependent manner. 120 mV (vs.Ag/AgCl) is the oxidation potential of N-(3-tert-butylphenyl)-p-phenylenediamine as the label, and a correlation is observed between the culture supernatant concentration and the label-derived oxidation current. Further, the oxidation current value is significantly lowered as a result of heat treatment, thereby strongly indicating that this oxidation current is based on enzyme activity. This demonstrates that electrochemical measurement enables quantification of gingipain activity in the culture supernatant.

In the examples above, endotoxin, trypsin, and gingipain included in a culture supernatant of gingvalis were subjected to electrochemical measurement using a label based on the phenylenediamine-type compound. While all of clotting enzyme, trypsin, and gingipain used for endotoxin measurement are proteases, a peptidase can also be measured. Further, a person skilled in the art would understand that the method of the present disclosure can be applied to various hydrolases such as phosphatases, e.g., alkaline phosphatase, esterase, and glycosidase and the like.

A method for measurement of alkaline phosphatase by the method of the present disclosure is described as follows. A compound of phosphoric acid bound to a label is used to perform electrochemical measurement of alkaline phosphatase. As the phosphoric acid-substrate-label compound, a compound in which phosphoric acid is bound to N-(3-tert-butylphenyl)-p-phenylenediamine, a compound in which phosphoric acid is bound to N-(3-isopropylphenyl)-p-phenylenediamine, a compound in which phosphoric acid is bound to N-(3-tert-butylphenyl)-p-phenylenediamine, or an analogous compound thereof may be used. For example, 10 µl of a solution of 1 mM phosphoric acid-N-(3-tert-butylphenyl)-p-phenylenediamine, 10 µl of a solution of alkaline phosphatase at a given concentration (0, 1, 2, 5, 10, or 20 U/l), and 80 µl of 0.1 mol/l carbonate buffer (pH 9.8) containing 2.0 mmol/l magnesium chloride are mixed in a dry-heat-sterilized test tube, and the resultant is allowed to stand in an incubator at 37°C for 1 hour. Thereafter, cyclic voltammetry is performed under the same conditions as in Example 2.

The correlation between the alkaline phosphatase concentration and the label-derived oxidation current is examined to quantify alkaline phosphatase via electrochemical measurement.

In the examples above, endotoxin, trypsin, and gingipain were subjected to electrochemical measurement mainly using a label derived from a phenylenediamine-type compound. The phenylenediamine-type compound used herein is a compound reported to be capable of being adsorbed onto an electrode. Therefore, a person skilled in the art understands that instead of a phenylenediamine-type compound, and by using a label derived from other compounds capable of being adsorbed onto an electrode, such as an aminoanthraquinone-based compound represented by Formula II, electrochemical measurement of endotoxin, trypsin, gingipain, and phosphatase can be performed.

### Industrial Applicability

The method for measurement of a trace substance according to the present disclosure can be used in the fields of, for example, fermentation, pharmaceutical, and biochemical studies.

Multiple documents including patent applications and manufacturers' instructions are cited herein. While the disclosures of such documents are not regarded as being related to patentability of the present disclosure, such disclosures are incorporated herein by reference in their entirety. More specifically, all the cited documents are incorporated herein by reference as in the case in which each of the documents is specifically and individually indicated to be incorporated herein by reference.

All publications, patents and patent applications cited herein are incorporated herein by reference in their entirety.

## Claims

1. A method of electrochemical measurement comprising using a substrate-label compound labeled with a label, wherein the label is used to convert a substrate into a substrate-label compound suitable for electrochemical measurement by being bound to the substrate, and wherein the label is capable of being adsorbed onto an electrode.

2. The method of claim 1, wherein the label is released from the substrate-label compound by the action of a hydrolase and the released label is measured electrochemically.

3. The method of measurement of claim 1 or 2 for measuring the hydrolase activity of a specimen or a substance that activates a hydrolase, which may be contained in the specimen.

4. The method according to any one of claims 1 to 3, wherein the substance that activates a hydrolase comprises endotoxin or (1→3)-β-D-glucan.

5. The method according to any one of claims 1 to 4, wherein the hydrolase is Factor C, Factor B, Factor G, and/or Proclotting enzyme of horseshoe crab, trypsin, gingipain, or phosphatase.

6. The method according to any one of claims 1 to 5, wherein the label is a phenylenediamine-type compound having a structure shown in Formula I: 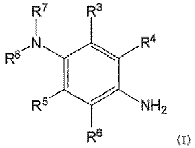 wherein
R⁷ represents hydrogen or linear or branched C₁₋₆ alkyl, C₁₋₆ alkenyl, C₁₋₆ alkynyl, C₃₋₉ cycloalkyl, phenyl, 1-naphthyl, 2-naphthyl, anthracenyl, or phenanthrenyl, which may be optionally substituted with one or more X;
R³, R⁴, R⁵, and R⁶ each independently represent hydrogen or linear or branched C₁₋₆ alkyl, C₁₋₆ alkenyl, C₁₋₆ alkynyl, C₁₋₆ alkoxy, halo, nitro, cyano, carboxy, sulfone, or amino, which may be optionally substituted with one or more Y; and
R⁸ is selected from the group consisting of phenyl, 1-naphthyl, 2-naphthyl, anthracenyl, and phenanthrennyl, which may be optionally substituted with one or more X,
wherein X represents linear or branched C₁₋₆ alkyl, C₁₋₆ alkenyl, C₁₋₆ alkynyl, C₁₋₆ alkoxy, anilino, phenoxy, halo, hydroxy, nitro, carboxy, cyano, sulfone, or amino, which may be optionally substituted with a substituent selected from the group consisting of halo, amino, cyano, carboxy, carbonyl, alkoxy, and sulfone, and
Y is selected from the group consisting of halo, amino, cyano, carboxy, carbonyl, alkoxy, and sulfone, or
an aminoanthraquinone-based compound having a structure shown in Formula II:

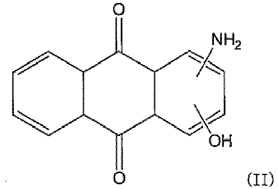

7. The method of claim 6, wherein the phenylenediamine-type compound shown in Formula I is a compound selected from the group consisting of: 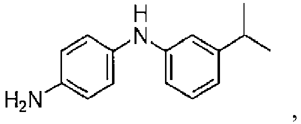 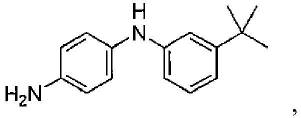 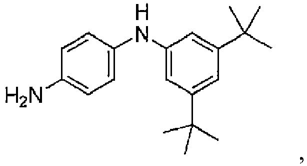 and 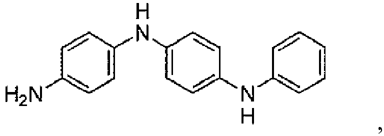 or
the aminoanthraquinone-based compound shown in Formula (II) is 1-amino-4-hydroxyanthraquinone having a structure shown in the following formula: 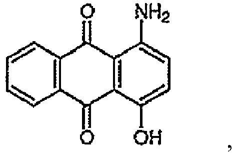
2-amino-3-hydroxyanthraquinone having a structure shown in the following formula: 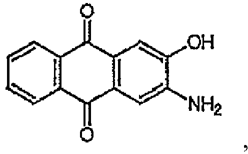 or
a salt, an anhydride, or a solvate thereof.

8. A reagent for electrochemical measurement of hydrolase activity or a substance that activates a hydrolase said reagent comprising a substrate-label compound, wherein a label in the substrate-label compound is capable of being adsorbed onto an electrode.

9. A composition for measurement of hydrolase activity or a substance that activates a hydrolase, said composition comprising the reagent of claim 8.

10. An electrode comprising the reagent of claim 8 or the composition of claim 9.

11. An electrochemical measurement system, wherein the electrode of claim 10 is used.

12. The reagent of claim 8, the composition of claim 9, the electrode of claim 10, or the system of claim 11, wherein the label is a phenylenediamine-type compound having a structure shown in Formula I: 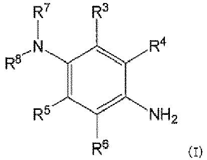 wherein
R⁷ represents hydrogen or linear or branched C₁₋₆ alkyl, C₁₋₆ alkenyl, C₁₋₆ alkynyl, C₃₋₉ cycloalkyl, phenyl, 1-naphthyl, 2-naphthyl, anthracenyl, or phenanthrenyl, which may be optionally substituted with one or more X;
R³, R⁴, R⁵, and R⁶ each independently represent hydrogen or linear or branched C₁₋₆ alkyl, C₁₋₆ alkenyl, C₁₋₆ alkynyl, C₁₋₆ alkoxy, halo, nitro, cyano, carboxy, sulfone, or amino, which may be optionally substituted with one or more Y; and
R⁸ is selected from the group consisting of phenyl, 1-naphthyl, 2-naphthyl, anthracenyl, and phenanthrennyl, which may be optionally substituted with one or more X,
wherein X represents linear or branched C₁₋₆ alkyl, C₁₋₆ alkenyl, C₁₋₆ alkynyl, C₁₋₆ alkoxy, anilino, phenoxy, halo, hydroxy, nitro, carboxy, cyano, sulfone, or amino, which may be optionally substituted with a substituent selected from the group consisting of halo, amino, cyano, carboxy, carbonyl, alkoxy, and sulfone, and
Y is selected from the group consisting of halo, amino, cyano, carboxy, carbonyl, alkoxy, and sulfone, or
an aminoanthraquinone-based compound having a structure shown in Formula II:

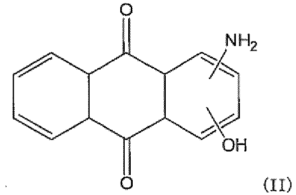

13. The reagent, composition, electrode, or system of claim 12, wherein the phenylenediamine-type compound shown in Formula I is a compound selected from the group consisting of: 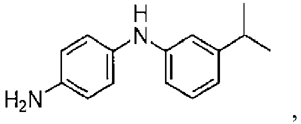 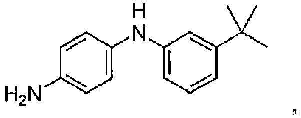 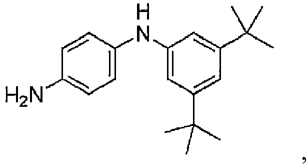 and 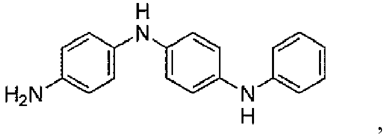 or
the aminoanthraquinone-based compound shown in Formula (II) is 1-amino-4-hydroxyanthraquinone having a structure shown in the following formula: 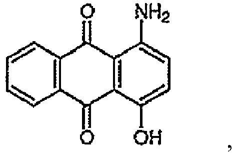
2-amino-3-hydroxyanthraquinone having a structure shown in the following formula: 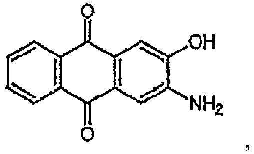 or
a salt, an anhydride, or a solvate thereof.
